# EUROPÄISCHE PATENTSCHRIFT

(11) **EP 0 491 138 B1**
(45) Veröffentlichungstag und Bekanntmachung des Hinweises auf die Patenterteilung: **10.01.1996**
(21) Anmeldenummer: 91118011.5
(22) Anmeldetag: 23.10.1991
(51) Int. Cl.: A61B 17/58

(54) **Vorrichtung zur Fixation von Knochenbrüchen**
Device for holding broken bones in fixed position
Dispositif de fixation d'os cassés

(30) Priorität: 17.12.1990 CH 3989/90
(43) Veröffentlichungstag der Anmeldung: 24.06.1992
(73) Patentinhaber: Synthes AG, Chur, CH-7002 Chur (CH)
(72) Erfinder: Frigg, Robert, CH-7270 Davos-Platz (CH)
(74) Vertreter: Lusuardi, Werther Giovanni, Dr.

(56) Entgegenhaltungen:
- EP-A- 0 382 256
- EP-A- 0 411 273
- DE-A- 1 800 150
- US-A- 2 441 765
- US-A- 3 107 666
- US-A- 3 256 877
- US-A- 4 103 683
- US-A- 4 733 654

## Beschreibung

Die Erfindung bezieht sich auf eine Vorrichtung zur Fixation von Knochenbrüchen in Gelenksnähe, gemäss der Gattung des Patentanspruchs 1.

Vorrichtungen dieser Gattung, welche gemeinhin auch als Winkelplatten bekannt sind, werden vorwiegend im Bereich der proximalen und distalen Femurfrakturen, sowie an der proximalen Tibia implantiert.

Aus der US-PS 3,025,853 MASON ist beispielsweise eine solche Winkelplatte bekannt, welche aus einer in den Oberschenkelhals einzuführenden Plattenklinge besteht, die in einem vorgegebenen Winkel fest mit einer am Femurschaft zu fixierenden Seitenplatte verbunden ist. Die Plattenklinge wird unter einem vorgegebenen Winkel in den Knochen eingeschlagen, wodurch das gelenknahe Knochenfragment gefasst wird. Die Seitenplatte kommt, nach erfolgtem vollständigen Einschlagen der Plattenklinge in den Oberschenkelhals, lateral am proximalen Femurschaft zu liegen und wird dort mit einer mehr oder weniger grossen Anzahl von Knochenschrauben fixiert.

Die Vorteile, welche mit dieser bekannten Winkelplattenfixation erzielt werden können, sind vor allem biomechanischer Art. Dank ihrer grossen Abstützfläche ermöglicht die Plattenklinge eine optimale Abstützung im spongiösen Bereich des gelenknahen Knochenfragmentes.
Die Hauptnachteile dieser Winkelplatte liegen in deren Handhabung begründet. Durch die einstückige Konstruktion muss der Orientierung der Plattenklinge höchste Beachtung geschenkt werden, da sonst die Längsachse der Seitenplatte nicht mit der Längsachse des Röhrenknochens übereinstimmt. Eine weitere Randbedingung ist durch die Anatomie des zu fixierenden Knochens gegeben; dabei handelt es sich um die Richtung, in der die Plattenklinge (z.B. am proximalen Femurkopf) von lateral nach medial eingeschlagen werden kann, ohne den Femurhals seitlich (gegen anterior oder posterior) zu durchstossen. Sind die drei genannten winkelmässigen Relationen durch den Chirurgen definiert worden, so muss die exakte Eintrittsstelle der Plattenklinge bestimmt werden. Diese Eintrittsstelle ergibt sich grundsätzlich aus den drei obengenannten Winkelbeziehungen. In der Praxis beobachtet man oft Abweichungen gewollter oder ungewollter Art. Diese Abweichungen sind, sofern sie ungewollt entstanden sind, äusserst schwer, wenn nicht unmöglich zu korrigieren. Der Grund dafür ist der, dass Abweichungen erst nach erfolgter Implantation der Plattenklinge sichtbar werden. Da die Dimension der Plattenklinge, bei der am häufigsten auftretenden Anwendung am proximalen Femur, den Querschnitt des Femurhalses fast vollständig ausfüllt, ist eine Korrektur nur bedingt möglich und führt zu einer instabilen Fixation, durch den nun zu grossen Sitz der Plattenklinge.
Die Implantation einer solchen bekannten Winkelplatte bedingt somit eine hervorragende dreidimensionale Vorstellungskraft des Chirurgen im Bereich der Anatomie, sowie eine sorgfältige und damit zeitaufwendige präoperative Planung.

Aus der US-A 2 441 765 HOPKINS ist ein Schenkelhalsnagel mit einem dreikantigen Nagel bekannt. Ein solcher dreikantiger Nagel weist wegen seines Profils im wesentlichen über seine gesamte Länge eine gleichbleibende Steifigkeit auf, was für den beabsichtigten Zweck nachteilig ist. Wenn man einen solchen Schenkelhalsnagel mit einem Marknagel kombiniert, so verlangt dies - wiederum wegen des dreikantigen Profils - eine übermässig grosse Öffnung im Marknagel (eine Querschnittsvergrösserung des Marknagels scheidet aus anatomischen Gründen aus), was die mechanische Festigkeit des Marknagels erheblich beeinträchtigt.

Aufgrund der bei den bekannten Winkelplatten auftretenden Schwierigkeiten sind denn auch viele Chirurgen auf bewegliche Hüft- oder Kondylenschrauben ausgewichen. Diese haben in Bezug auf die Handhabung bei der Implantation wesentliche Vorteile gegenüber der Winkelplatte. Einer der grössten Vorteile stellt dabei die Möglichkeit einer "Vorsondierung" der Schraubenlage mit Hilfe eines Führungsdrahtes dar. Dieser Führungsdraht wird unter Röntgenkontrolle in das zu fixierende Knochenfragment geführt.
Da auch diese bekannten bewegliche Hüftschrauben unter einem fixen Winkel in die Hülse der Seitenplatte eingeführt werden, muss auch hier sehr genau gearbeitet werden, was durch Verwendung einer Zielvorrichtung und des Führungsdrahtes geschieht; sobald der Führungsdraht optimal in dem zu fixierenden Knochen liegt, wird die Bohrung für die bewegliche Hüftschraube über dem liegenden Führungsdraht durchgeführt. Nach erfolgtem Eindrehen der beweglichen Hüftschraube kann die Seitenplatte auf den Schraubenschaft geschoben werden. Einige bekannte Schraubentypen besitzen Längsnuten am Schraubenschaft, die mit den entsprechenden Nutensteinen im Gleitschaft (Hülse) der Seitenplatte übereinstimmen müssen; dies ist jedoch kein Problem, da die bewegliche Hüftschraube, bei fehlender Übereinstimmung weiter eingedreht oder ausgedreht werden kann.
Der Nachteil der beweglichen Hüftschraube liegt hingegen in der oft ungenügenden Rotationsstabilität und Fixation, sowie im zu massiven Implantatquerschnitt. Betrachtet man die Belastungen, welche auf das Implantat wirken, so erkennt man, dass eine bewegliche Hüftschraube der Winkelplatte unterlegen ist. Das Gleiche gilt auch für die Fixation von Frakturen im Bereich der Femurkondylen.

Hier will die Erfindung Abhilfe schaffen. Der Erfindung liegt die Aufgabe zugrunde, die biomechanischen Vorteile der Winkelplatte mit der einfachen Operationstechnik der beweglichen Hüftschraube zu kombinieren.

Die Erfindung löst die gestellte Aufgabe mit einer Vorrichtung, welche die Merkmale des Anspruchs 1 aufweist.

Weitere vorteilhafte Ausgestaltungen der Erfindung sind in den abhängigen Ansprüchen gekennzeichnet.

Ausführungsbeispiele der Erfindung werden nachstehend anhand der Zeichnung näher erläutert.

Es zeigen:
Fig. 1 teils in einer Seitenansicht, teils in einem senkrechten Schnitt eine erfindungsgemässe Vorrichtung bestehend aus einer unter einem stumpfen Winkel fest mit der Seitenplatte verbindbaren, in einen Marknagel eingeführten Plattenklinge;
Fig. 2 in einer Ansicht von lateral den Marknagel der Vorrichtung nach Fig. 1;
Fig. 3 teils in einer Seitenansicht, teils in einem senkrechten Schnitt den im proximalen Femur implantierten Marknagel der Vorrichtung nach Fig. 1;
Fig. 4 teils in einer Seitenansicht, teils in einem senkrechten Schnitt den im proximalen Femur implantierten Marknagel mit der durch seinen Schlitz hindurchgeführten Plattenklinge der Vorrichtung nach Fig. 1;
Fig. 5 teils in einer Seitenansicht, teils in einem senkrechten Schnitt die vollständig zusammengesetzte im proximalen Femur implantierte Vorrichtung nach Fig. 1;
Fig. 6 teils in einer Seitenansicht, teils in einem senkrechten Schnitt eine modifizierte Seitenplatte für eine erfindungsgemässe Vorrichtung, bei welcher die Plattenklinge unter einem rechten Winkel fest mit der Seitenplatte verbindbar ist;
Fig. 7 in einer Ansicht die Plattenklinge gemäss Fig. 6 in Richtung ihrer Längsachse von der Position A-A aus gesehen;
Fig. 8 in einer Ansicht die Plattenklinge gemäss Fig. 6 in Richtung ihrer Längsachse von der Position B-B aus gesehen;
Fig. 9 teils in einer Seitenansicht, teils in einem senkrechten Schnitt eine modifizierte Seitenplatte mit Plattenklinge für eine erfindungsgemässe Vorrichtung, bei welcher die Plattenklinge unter einem rechten Winkel teleskopisch mit der Seitenplatte verbindbar ist;
Fig. 10 teils in einer Seitenansicht, teils in einem senkrechten Schnitt die Plattenklinge nach Fig. 9;
Fig. 11 in einer Seitenansicht eine modifizierte Seitenplatte mit Plattenklinge für eine erfindungsgemässe Vorrichtung, bei welcher die Plattenklinge unter einem stumpfen Winkel teleskopisch mit der Seitenplatte verbindbar ist; und
Fig. 12 in einer Seitenansicht die mit ihrem Schaft teleskopisch gleitend in der Gleithülse der Seitenplatte gelagerte Plattenklinge nach Fig. 11.

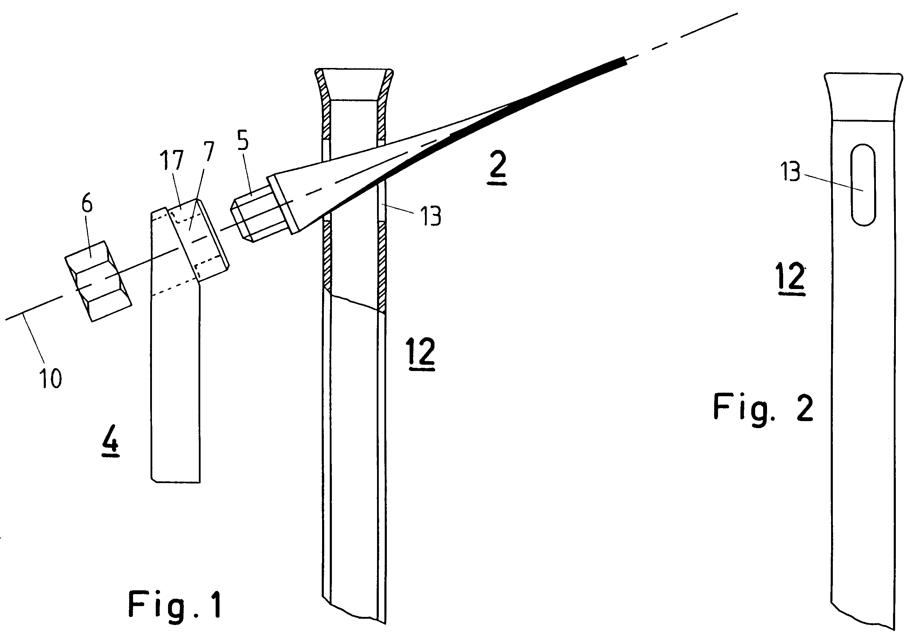

Wie in Fig. 1 gezeigt besteht die erfindungsgemässe Vorrichtung im wesentlichen aus einer Seitenplatte 4, einer daran lösbar befestigbaren Plattenklinge 2 und einem die Plattenklinge 2 aufnehmenden Marknagel 12.

Die Plattenklinge 2 weist einen endständigen, zylinderförmigen Gewindeteil 5 auf, der in eine entsprechende Bohrung 7 im oberen Teil der Seitenplatte 4 von der einen Plattenseite eingeführt und mit einer Mutter 6 von der anderen Plattenseite aus lösbar daran fixiert werden kann. Um die Vorrichtung kompakt zu halten ist die Bohrung 7 in der Seitenplatte 4 derart gestaltet, dass die Mutter 6 darin versenkbar ist.

Wie in Fig. 2 dargestellt weist der Marknagel 12 in seinem oberen Teil einen durchgehenden Schlitz 13 zur Aufnahme der Plattenklinge 2 auf.

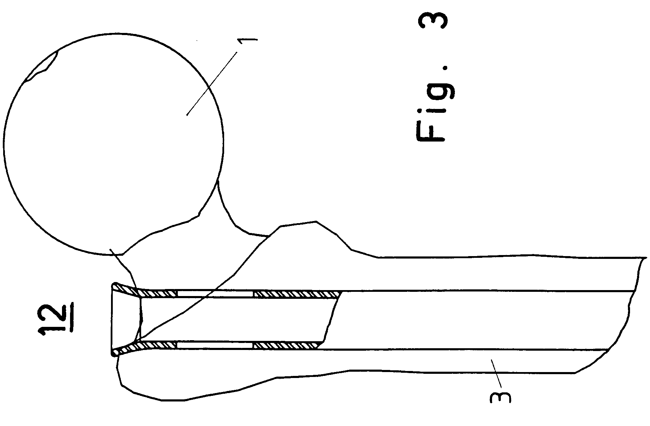

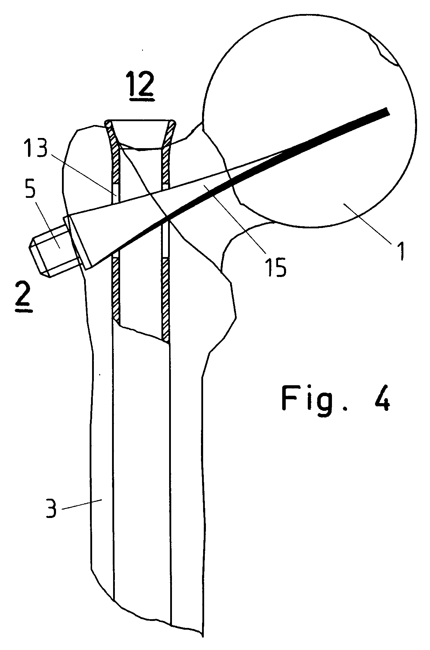

Nachstehend wird nun kurz die Implantation der erfindungsgemässen Vorrichtung in den Femur beschrieben:
In einem ersten operativen Schritt erfolgt die Implantation des Marknagels 12 im Femurschaft 3, wie in Fig. 3 dargestellt. Der Schlitz 13 wird dabei in die medio-laterale Richtung gedreht. Fig. 4 zeigt, wie in einem zweiten Schritt die Plattenklinge 2 mit ihrer schraubenförmig gewundenen Klinge 15 durch den Schlitz 13 des Marknagels 12 hindurch geführt wird. In einem dritten Schritt wird der endständige, zylinderförmige Gewindeteil 5 der Plattenklinge 2 in die entsprechende Bohrung des Aufnahmeteils 17 im oberen Teil der Seitenplatte 4 eingeführt und mit einer Mutter 6 daran fixiert. Schliesslich wird in bekannter Weise die Seitenplatte 4 mittels einer Anzahl (zeichnerisch nicht dargestellter) Knochenschrauben am Femurschaft fixiert.

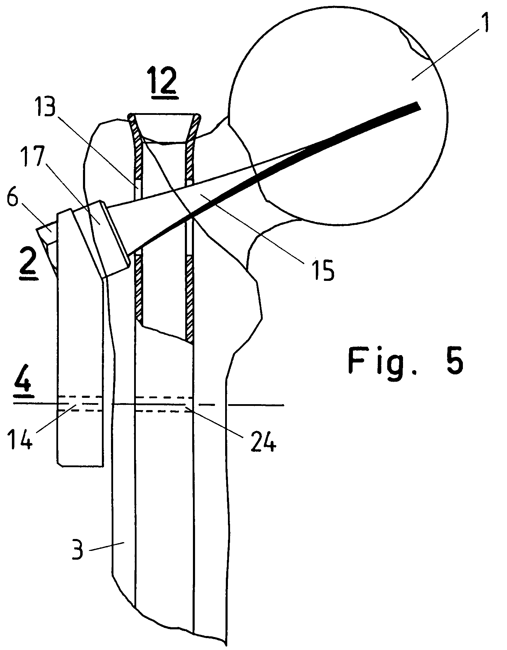

Wie in Fig. 5 dargestellt kann der Marknagel 12 im seinem mittleren Bereich Bohrungen 24 aufweisen, welche mit den Bohrungen 14 der Seitenplatte 4 fluchten. Mittels geeigneter Knochenschrauben, welche durch beide Bohrungen 14 und 24 hindurchgeführt werden, kann eine Verankerung der Seitenplatte 4 durch die nahe Kortikalis des Femurschaft 3 hindurch direkt am Marknagel 12 realisiert werden, wodurch sich eine erhöhte Stabilität der erfindungsgemässen Vorrichtung 2,4,12 ergibt. Die schraubenförmig gewundenen Klinge 15 der Plattenklinge 2 wird dabei in beweglicher Weise von der bodenseitigen Wandung des Schlitzes 13 im Marknagel 12 unterstützt, so dass eine winkelmässige und/oder longitudinale Positionierung der schraubenförmig gewundenen Klinge 15 mit Hilfe der durch die Bohrungen 14 und 24 hindurchgeführten Knochenschrauben möglich ist.

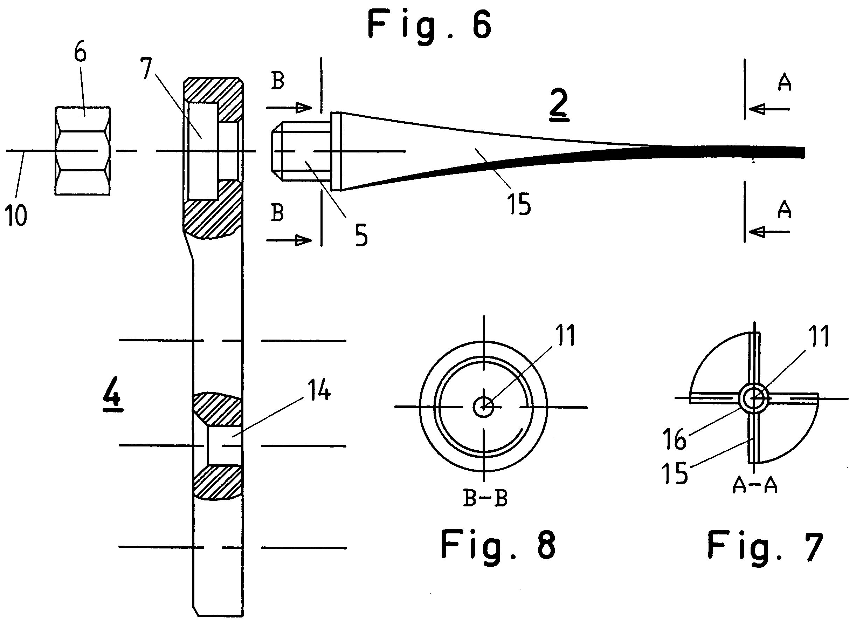

Die Plattenklinge 2 ist, wie in den Fig. 6 - 8 im Detail dargestellt, als schraubenförmig um ihre Längsachse 10 gewundene Klinge 15 ausgebildet, vorzugsweise in kontinuierlicher, einen Drehwinkelbereich von annähernd 90° umfassenden Weise. Die Plattenklinge 2 ist mit einer zentralen, in Richtung der Längsachse 10 verlaufende Bohrung 11 versehen. Die Bohrung 11 erlaubt dem Chirurgen die Plattenklinge 2 über einen Führungsdraht zu plazieren, wie dies bei den herkömmlichen dynamischen Hüftschrauben bekannt ist. Das Profil der Plattenklinge 2 besitzt eine Verdickung 16 um die Bohrung 11 herum, um die flügelförmigen Lamellen der schraubenförmig gewundenen Klinge 15 tragen zu können. Die flügelförmigen Lamellen der schraubenförmig gewundenen Klinge 15 können sich über die gesamte Länge der Plattenklinge 2, oder nur über einen Teil derselben erstrecken. Je nach Anwendung kann statt der spiralförmigen Geometrie der Plattenklinge 2 auch eine ebene (zeichnerisch nicht dargestellte) Konfiguration gewählt werden.

In der Vorrichtung nach Fig. 1 steht die Plattenklinge 2 nicht in einem rechten Winkel zur Seitenplatte 4, sondern in einem anatomisch bedingten, stumpfen Winkel. Zweckmässigerweise weist bei dieser Ausführungsform die Seitenplatte 4 einen innerhalb eines gewissen Winkelbereiches von vorzugsweise 90°- 150° schwenkbaren und beliebig fixierbaren, die Bohrung 7 enthaltenden, Aufnahmeteil 17 auf, dessen konstruktive Details zeichnerisch nicht dargestellt sind.

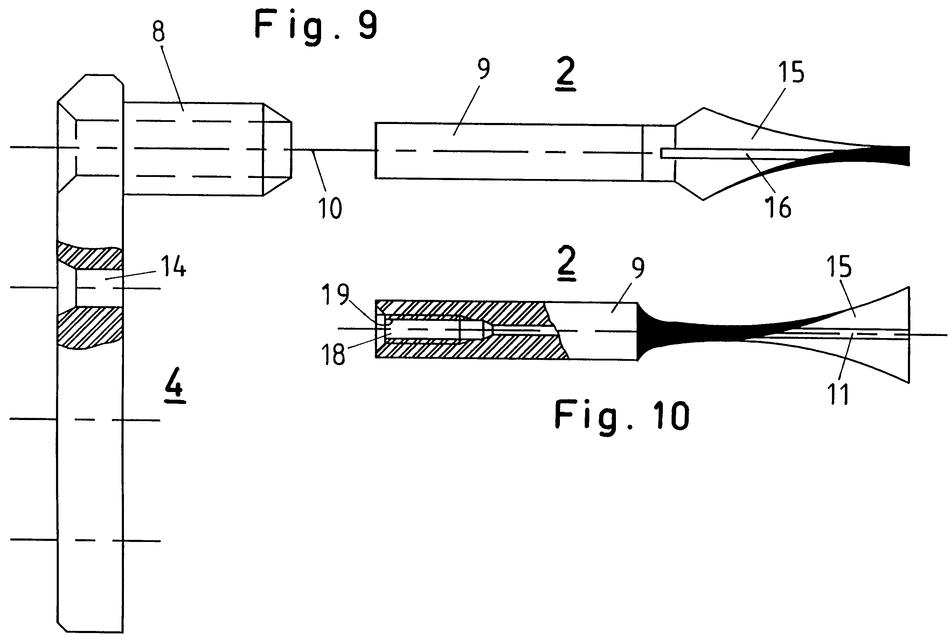

Die Ausführungsform gemäss Fig. 1 zeigt eine zwar lösbare, aber nach erfolgter Implantation feste Verbindung zwischen Plattenklinge 2 und Seitenplatte 4. Erfordert die Indikation jedoch wie bei einer dynamischen Hüftschraube eine gleitende Verbindung zwischen Plattenklinge 2 und Seitenplatte 4 so kann das laterale Ende der Plattenklinge 2, wie in den Fig. 9 und 10 dargestellt in Form eines zylindrischen Schaftes 9 ausgebildet sein, welcher in eine dazu korrespondierende, am oberen Ende der Seiteplatte 4 angebrachten Gleithülse 8 einführbar und darin teleskopisch bewegbar ist. Die Gleithülse 8 muss dabei so dimensioniert sein, dass der sich lateral befindliche Schaft 9 der Plattenklinge 2 in ihr frei gleiten kann. Auch bei dieser teleskopierbaren Ausführungsform ist die Plattenklinge 2 als schraubenförmig gewundenen Klinge 15 ausgebildet, welche eine zentrale Bohrung 11 aufweist.

Wie in Fig. 10 dargestellt kann der Schaft 9 der Plattenklinge 2 ausserdem mit einer axialen Bohrung 18 mit Innengewinde 19 versehen sein um eine (zeichnerisch nicht dargestellte) Kompressionsschraube aufnehmen zu können.

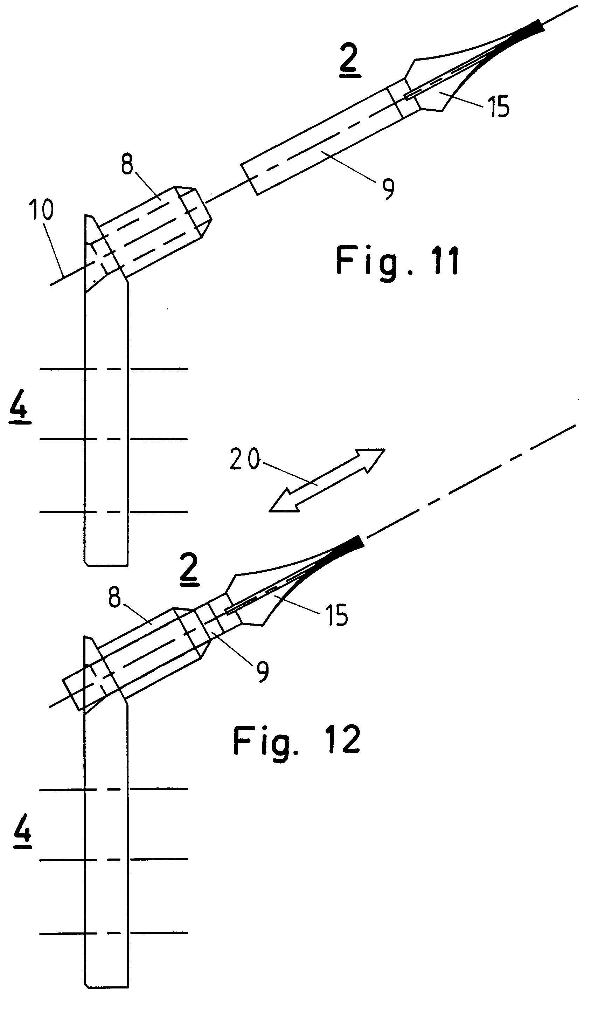

In Fig. 11 ist eine weitere Ausführungsform der Verbindung zwischen Seitenplatte 4 und Plattenklinge 2 dargestellt, bei welcher analog zur Vorrichtung gemäss Fig. 1 die Plattenklinge 2 unter einem stumpfen Winkel teleskopisch mit der Seitenplatte 4 verbindbar ist. Auch hier ist die Gleithülse 8 vorzugsweise schwenkbar und beliebig fixierbar in der Seitenplatte 4 gelagert um verschiedene Winkelstellungen realisieren zu können. In Fig. 12 ist dargestellt, wie der Schaft 9 der Plattenklinge 2 in der Gleithülse 8 der Seitenplatte 4 in Richtung des Pfeiles 20 teleskopisch gleitend gelagert ist.

## Patentansprüche

1. Vorrichtung zur Fixation von Knochenbrüchen in Gelenksnähe, mit einer zur Implantation in das gelenknahe Knochenfragment (1) bestimmten Plattenklinge (2) und einer zur Befestigung am Schaft des gelenkfernen Röhrenknochens (3) bestimmten Seitenplatte (4), dadurch gekennzeichnet, dass
A) die Plattenklinge (2) an einem ihrer beiden Enden mit der Seitenplatte (4) lösbar verbindbar ist;
B) die Vorrichtung einen Marknagel (12) umfasst, der in seinem oberen Teil in Längsrichtung einen durchgehenden Schlitz (13) zur beweglichen Aufnahme der Plattenklinge (2) aufweist; und
C) der Marknagel (12) im implantierten Zustand zwischen der Verbindungsstelle von Seitenplatte (4) mit der Plattenklinge (2) und dem anderen, freien Ende der Plattenklinge (2) angeordnet ist.

2. Vorrichtung nach Anspruch 1, dadurch gekennzeichnet, dass Plattenklinge (2) und Seitenplatte (4) in zueinander fester Relation, vorzugsweise in Form einer Gewindeverbindung (5,6,7), lösbar miteinander verbindbar sind.

3. Vorrichtung nach Anspruch 1, dadurch gekennzeichnet, dass Plattenklinge (2) und Seitenplatte (4) in winkelmässig zueinander einstellbarer Weise, lösbar miteinander verbindbar sind.

4. Vorrichtung nach Anspruch 3, dadurch gekennzeichnet, dass die Seitenplatte (4) eine Gleithülse (8) aufweist und die Plattenklinge (2) einen teleskopisch in die Hülse (8) einführbaren Schaft (9) aufweist.

5. Vorrichtung nach Anspruch 2 oder 4, dadurch gekennzeichnet, dass die Gewindeverbindung (5,6,7) oder die Gleithülse (8) relativ zur Seitenplatte (4) winkelmässig schwenkbar und fixierbar angeordnet ist.

6. Vorrichtung nach Anspruch 5, dadurch gekennzeichnet, dass Plattenklinge (2) und Seitenplatte (4) unter einem wählbaren Winkel, vorzugsweise im Bereich zwischen 90°- 150°, zueinander verbindbar sind.

7. Vorrichtung nach einem der Ansprüche 1 bis 6, dadurch gekennzeichnet, dass die Plattenklinge (2) eine zentrale, in Richtung der Längsachse (10) verlaufende Bohrung (11) aufweist.

8. Vorrichtung nach einem der Ansprüche 1 bis 7, dadurch gekennzeichnet, dass die Seitenplatte (4) mindestens eine Bohrung (14) aufweist zur Aufnahme einer Knochenschraube mit der die Seitenplatte am Schaft des gelenkfernen Röhrenknochens (3) fixierbar ist.

9. Vorrichtung nach einem der Ansprüche 1 - 8, dadurch gekennzeichnet, dass der Marknagel (12) mindestens eine Bohrung (24) aufweist, welche mit der Bohrung (14) der Seitenplatte (4) fluchtet, zur Aufnahme einer beide Bohrungen (14,24) durchdringenden Knochenschraube.

10. Vorrichtung nach einem der Ansprüche 1 bis 9, dadurch gekennzeichnet, dass die Plattenklinge (2) als schraubenförmig um ihre Längsachse (10) gewundene Klinge (15) ausgebildet ist, vorzugsweise in kontinuierlicher, einen Drehwinkelbereich von annähernd 90° umfassenden Weise.

11. Vorrichtung nach Anspruch 10, dadurch gekennzeichnet, dass die Klinge (15) derart geformt ist, dass ihr im Schlitz (13) des Marknagels (12) zu liegende Bereich seine grösste Ausdehnung im wesentlichen parallel zur Längsachse des Marknagels (12) und der am freien Ende der Klinge (15) liegende Bereich seine grösste Ausdehnung im wesentlichen quer zur Längsachse des Marknagels (12) orientiert hat.

## Claims

1. Device for fixing fractures in the region of joints, having a plate blade (2) intended for implantation in the bone fragment (1) close to the joint and a side plate (4) intended for fastening to the shaft of the long bone (3) away from the joint, characterized in that
A) the plate blade (2) can be releasably connected at one of its two ends to the side plate (4);
B) the device comprises a medullary pin (12) which has in its upper part in the longitudinal direction a continuous slot (13) to movably receive the plate blade (2); and
C) the medullary pin (12) in the implanted state is arranged between the connection point of the side plate (4) with the plate blade (2) and the other free end of the plate blade (2).

2. Device according to claim 1, characterized in that the plate blade (2) and the side plate (4) can be releasably connected to each other in fixed relation to each other, preferably in the form of a threaded connection (5, 6, 7).

3. Device according to claim 1, characterized in that the plate blade (2) and the side plate (4) can be releasably connected to each other in an adjustable manner in terms of their angles in relation to each other.

4. Device according to claim 3, characterized in that the side plate (4) has a slide bush (8) and the plate blade (2) has a shaft (9) which can be telescopically inserted into the bush (8).

5. Device according to claim 2 or 4, characterized in that the threaded connection (5, 6, 7) or the slide bush (8) is arranged so that it can be rotated in angular manner relative to the side plate (4) and it can be fixed.

6. Device according to claim 5, characterized in that the plate blade (2) and the side plate (4) can be connected to each other at a selectable angle, preferably in the range between 90° to 150°.

7. Device according to one of claims 1 to 6, characterized in that the plate blade (2) has a central bore (11) extending in the direction of the longitudinal axis (10).

8. Device according to one of claims 1 to 7, characterized in that the side plate (4) has at least one bore (14) to receive a bone screw with which the side plate can be fixed to the shaft of the long bone (3) away from the joint.

9. Device according to one of claims 1 to 8, characterized in that the medullary pin (12) has at least one bore (24) which is in alignment with the bore (14) of the side plate (4), to receive a bone screw penetrating both bores (14, 24).

10. Device according to one of claims 1 to 9, characterized in that the plate blade (2) is constructed as a blade (15) turned in screw-like manner about its longitudinal axis (10), preferably in a continuous manner enclosing an angle of rotation range of approximately 90°.

11. Device according to claim 10, characterized in that the blade (15) is formed in such a way that its region which is to lie in the slot (13) of the medullary pin (12) has its greatest extent oriented substantially parallel to the longitudinal axis of the medullary pin (12) and the region lying at the free end of the blade (15) its greatest extent substantially at right angles to the longitudinal axis of the medullary pin (12).

## Revendications

1. Dispositif pour la réduction de fractures osseuses à proximité d'articulations, comportant une lame de plaque (2) destinée à l'implantation dans le fragment osseux (1) proche de l'articulation, et une plaque latérale (4) destinée à la fixation sur la tige de l'os tubulaire (3) éloigné de l'articulation, caractérisé en ce que
A) la lame de plaque (2) peut être reliée de manière libérable à la plaque latérale (4) par une de ses deux extrémités;
B) le dispositif comporte un clou médullaire (12) qui présente dans sa partie supérieure une fente traversante (13) s'étendant dans la direction longitudinale, pour la réception mobile de la lame de plaque (2); et
C) à l'état implanté, le clou médullaire (12) est disposé entre le point d'assemblage de la plaque latérale (4) avec la lame de plaque (2) et l'autre extrémité libre de la lame de plaque (2).

2. Dispositif selon la revendication 1, caractérisé en ce que la lame de plaque (2) et la plaque latérale (4) peuvent être assemblées l'une avec l'autre de manière libérable dans une relation mutuelle fixe, de préférence sous la forme d'un assemblage fileté (5, 6, 7).

3. Dispositif selon la revendication 1, caractérisé en ce que la lame de plaque (2) et la plaque latérale (4) peuvent être assemblées l'une avec l'autre de manière libérable, d'une manière mutuellement réglable angulairement.

4. Dispositif selon la revendication 3, caractérisé en ce que la plaque latérale (4) présente une douille coulissante (8), et la lame de plaque (2) présente une tige (9) pouvant être enfoncée télescopiquement dans la douille (8).

5. Dispositif selon la revendication 2 ou 4, caractérisé en ce que l'assemblage fileté (5, 6, 7) ou la douille coulissante (8) sont disposés de manière à pouvoir être immobilisés et à pouvoir pivoter angulairement par rapport à la plaque latérale (4).

6. Dispositif selon la revendication 5, caractérisé en ce que la lame de plaque (2) et la plaque latérale (4) peuvent être assemblées l'une avec l'autre sous un angle sélectionnable, de préférence dans la plage s' étendant entre 90° et 150°.

7. Dispositif selon l'une quelconque des revendications 1 à 6, caractérisé en ce que la lame de plaque (2) présente un alésage central (11) s'étendant dans la direction de l'axe longitudinal (10).

8. Dispositif selon l'une quelconque des revendications 1 à 7, caractérisé en ce que la plaque latérale (4) présente au moins un alésage (14), pour la réception d'une vis osseuse par laquelle la plaque latérale peut être fixée sur la tige de l'os tubulaire (3) éloigné de l'articulation.

9. Dispositif selon l'une quelconque des revendications 1-8, caractérisé en ce que le clou médullaire (12) présente au moins un alésage (24) qui est aligné sur l'alésage (14) de la plaque latérale (4), pour la réception d'une vis osseuse traversant les deux alésages (14, 24).

10. Dispositif selon l'une quelconque des revendications 1 à 9, caractérisé en ce que la lame de plaque (2) est configurée comme lame torsadée en forme de vis (15) autour de son axe longitudinal (10), de préférence de manière continue et sur une plage angulaire de rotation d'environ 90°.

11. Dispositif selon la revendication 10, caractérisé en ce que la lame (15) est configurée de telle sorte que sa région qui doit être placée dans la fente (13) du clou médullaire (12) présente son extension la plus grande sous une orientation essentiellement parallèle à l'axe longitudinal du clou médullaire (12), et la région située à l'extrémité libre de la lame (15) présente sa plus grande extension dans une orientation essentiellement transversale par rapport à l'axe longitudinal du clou médullaire (12).
